# NEUE EUROPÄISCHE PATENTSCHRIFT

(11) **EP 1 110 536 B2**
(45) Veröffentlichungstag und Bekanntmachung des Hinweises auf die Entscheidung über den Einspruch: **30.12.2009**
(45) Hinweis auf die Patenterteilung: 12.06.2002
(21) Anmeldenummer: 00123819.5
(22) Anmeldetag: 02.11.2000
(51) Int. Cl.: A61K 8/18, A61K 8/99

(54) **Polymerkombination für Haarbehandlungsmittel**
Hair treating composition comprising a polymer combination
Composition de traitement capillaire comprenant une combinaison de polymères

(30) Priorität: 02.12.1999 DE 19957947
(43) Veröffentlichungstag der Anmeldung: 27.06.2001
(73) Patentinhaber: Wella Aktiengesellschaft, 64274 Darmstadt (DE)
(72) Erfinder: Birkel, Susanne, 95496 Glashütten (DE); Lede, Michael, 63225 Langen (DE)

(56) Entgegenhaltungen:
- EP-A- 0 841 060
- EP-A1- 0 521 666
- EP-A1- 0 524 346
- EP-A2- 0 640 334
- WO-A-96/19971
- WO-A-96/20694
- WO-A-98/22076
- WO-A1-95/00104
- FR-A1- 2 737 660
- FR-A1- 2 750 047
- FR-A1- 2 755 607
- US- - 4 521 404
- US- - 5 158 762
- Schreiben der ISP Deutschland GmbH an die Hans Schwartzkopf GmbH vom 15.12.1998
- 'ISP flexes its arm' SPC März 1998, Seite 61
- COSMETICS & TOILETRIES Bd. 103, Mai 1988, Seite 88
- J.A. WENNINGER ET AL: 'International Cosmetic Ingredient Dictionary', Bd. 1, teil 6TH ED 1995, THE COSMETIC, TOILETRY AND FRAGRANCE ASSOCIATION, WASHINGTON Seite 142
- KARLHEINZ SCHRADER: 'Grundlagen und Rezepturen der Kosmetika', 1989, HÜTHIG BUCH VERLAG GMBH, HEIDELBERG, ISBN 3-7785-1491-1 Artikel 'Beschreibung kosmetischer Grund-, Hilfs- und Wirkstoffe', Seite 81

## Beschreibung

Gegenstand der Erfindung ist eine Polymerkombination sowie ein Haarbehandlungsmittel mit einem Gehalt an mindestens einem Terpolymeren aus Vinylpyrrolidon, Vinylcaprolactam und einem basischen Acrylamidmonomer sowie mindestens einem Polymeren mit anionischen oder anionisierbaren Gruppen.

Um dem menschlichen Haar Festigung und Halt zu geben oder um eine erstellte Frisur zu stabilisieren, werden Haarbehandlungsmittel in Form von Festigerlotionen, Aerosol- und Non-Aerosolsprays, Festigerschäumen, Gelen etc. eingesetzt. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung mehr oder weniger gut die Haare in Form halten und festigen und die erstellte Frisur stabilisieren. Häufig ist damit aber ein starrer, unnatürlicher Griff des Haares verbunden und die Elastizität der Polymerfilme oder der polymervernetzten Frisur ist ungenügend.

Aus der WO 96/19971 sind Terpolymere aus Vinylpyrrolidon, Vinylcaprolactam und 3-(N-Dimethylaminopropyl)-methacrylamid bekannt sowie deren Verwendung in haarfestigenden Mitteln, insbesondere in Aerosol- und Pumpsprays. Diese Polymere sind besonders für einen Einsatz in wasserhaltigen Sprayrezepturen mit einem reduzierten Gehalt an leicht flüchtigen organischen Bestandteilen (low VOC-Sprays) geeignet. Die Polymere haben gute festigende Eigenschaften, verleihen dem Haar aber einen relativen rauhen und unelastischen Griff und eine relativ hohe Belastung.

Es bestand daher die Aufgabe, die filmbildenden und haarfestigenden Eigenschaften von polymerhaltigen Zubereitungen weiter zu verbessern und insbesondere die Elastizität von Polymerfilmen bzw. von polymerbehandelten Haaren zu steigern.

Es wurde nun gefunden, dass die Aufgabe gelöst wird durch eine Kombination aus zwei ausgewählten Polymeren bzw. durch ein Haarbehandlungsmittel, welches diese Polymerkombination enthält. Gegenstand der Erfindung ist eine Polymerkombination aus
(A) mindestens einem Terpolymeren aus Vinylpyrrolidon, Vinylcaprolactam und einem basischen Acrylamidmonomer und
(B) mindestens einem Polymeren mit anionischen oder anionisierbaren Gruppen, **dadurch gekennzeichnet, dass** das Polymer (B) ausgewählt ist aus vernetzten oder unvernetzten Vinylacetat/Crotonsäure Copolymeren, Vinylacetat/Crotonsäure/Vinylalkanoat Copolymeren, Terpolymeren aus Acrylsäure, Alkylacrylat und N-Alkylacrylamid, Copolymeren aus Methylvinylether und Maleinsäuremonoalkylestern und amphoteren Polymeren.

Ein weiterer Gegenstand der Erfindung ist ein Haarbehandlungsmittel mit einem Gehalt an der genannten Polymerkombination in einer geeigneten kosmetischen Grundlage. Die erfindungsgemäße Polymerkombination bewirkt eine Erhöhung, insbesondere eine synergistische Steigerung der Elastizität des Polymerfilms bzw. von polymerbehandelten Haaren.

Das Terpolymer (A) ist in dem erfindungsgemäßen Mittel vorzugsweise in einer Menge von 0,01 bis 20, besonders bevorzugt von 0,05 bis 10, ganz besonders bevorzugt von 0,1 bis 5 Gew.% und das anionische Polymer (B) in einer Menge von 0,01 bis 20, besonders bevorzugt von 0,05 bis 10, ganz besonders bevorzugt von 0,1 bis 5 Gew.% enthalten.

Geeignete Terpolymere (A) sind solche, bei denen das Acrylamidmonomer ausgewählt ist aus Dialkylaminoalkylmethacrylamid und Dialkylaminoalkylacrylamid, wobei die Alkylgruppen aus 1 bis 4 Kohlenstoffatomen bestehen. Besonders bevorzugt ist Dimethylaminopropylmethacrylamid. Die Herstellung eines derartigen Polymers wird in der WO 96/19971 beschrieben und ist unter der Bezeichnung Aquaflex® SF 40 (ISP) im Handel erhältlich (INCI-Bezeichnung: PVP/Vinyl Caprolactam/DMAPA Acrylates Copolymer).

Bei dem Polymer (B) kann es sich sowohl um sogenannte anionische Polymere handeln als auch um amphotere Polymere, welche neben anionischen oder anionisierbaren Gruppen auch basische Gruppen im Polymermolekül enthalten.

Unter anionisierbaren Gruppen werden Säuregruppen wie z.B. Carbonsäure-, Sulfonsäure- oder Phosphorsäuregruppen verstanden, welche mittels üblicher Basen wie z.B. organischer Amine oder Alkali- oder Erdalkalihydroxide deprotoniert werden können.

Die Polymere der Komponente (B) können teilweise oder vollständig mit einem basischen Neutralisationsmittel neutralisiert sein. Bevorzugt sind solche Mittel, in welchen im Polymer der Komponente (B) die sauren Gruppen zu 50 bis 100 %, besonders bevorzugt zu 70-100% neutralisiert sind. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH, KOH u.a..

Polymere mit Säuregruppen sind vernetzte oder unvernetzte Vinylacetat/Crotonsäure Copolymere (INCI-Bezeichnung: VA/Crotonates Copolymer), Vinylacetat/Crotonsäure/Vinylalkanoat Copolymere (INCI-Bezeichnungen: VA/Crotonates/Vinyl Propionate Copolymer, VA/Crotonates/Vinyl Neodecanoate Copolymer), Terpolymere aus Acrylsäure, Alkylacrylat und N-Alkylacrylamid, insbesondere Acrylsäure/Ethylacrylat/N-t-Butylacrylamid Terpolymere (INCI-Bezeichnung: Acrylates/Acrylamide Copolymer), Copolymere aus Methylvinylether und Maleinsäuremonoalkylestern (INCl-Bezeichnungen: Ethylester of PVM/MA Copolymer, Butylester of PVM/MA Copolymer).

Weitere, Polymere mit sauren Gruppen sind amphotere Polymere wie zum Beispiel Copolymere gebildet aus Alkylacrylamid, insbesondere Octylacrylamid, Alkylaminoalkylmethacrylat, insbesondere t-Butylaminoethylmethacrylate und zwei oder mehr Monomeren bestehend aus Acrylsäure, Methacrylsäure oder deren Ester (INCI-Bezeichnung: Octylacrylamide/Acrylates/Butylaminoethyl Methacrylate Copolymer). Weitere geeignete amphotere Polymere sind Copolymere, welche gebildet sind aus mindestens einer ersten Monomerart, welche quaternäre Amingruppen aufweist und mindestens einer zweiten Monomerart, welche Säuregruppen aufweist. Beispiele für derartige Copolymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43).

In einer weiteren bevorzugten Ausführungsform enthält das erfindungsgemäße Mittel zusätzlich 0,01 bis 15 Gewichtsprozent, vorzugsweise 0,5 bis 10 Gewichtsprozent mindestens eines synthetischen oder natürlichen nichtionischenfilmbildenden Polymers. Unter natürlichen Polymeren werden auch chemisch modifizierte Polymere natürlichen Ursprungs verstanden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Unter filmbildenden Polymeren werden erfindungsgemäß solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinylformamids. Weitere geeignete synthetische filmbildende, nichtionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol Copolymere, die beispielsweise unter den Handelsbezeichnungen Ucon® der Union Carbide vertrieben werden. Besonders bevorzugt sind Polyvinylpyrrolidon und Polyvinylpyrrolidon/Vinylacetat Copolymere.

Geeignete natürliche filmbildende Polymere sind z.B. Cellulosederivate, z.B. Hydroxypropylcellulose mit einem Molekulargewicht von 30.000 bis 50.000 g/mol, welche beispielsweise unter der Handelsbezeichnung Nisso SI® von der Firma Lehmann & Voss, Hamburg, vertrieben wird.

Das erfindungsgemäße Mittel wird bevorzugt in einem wässrigen, einem alkoholischen oder in einem wässrig-alkoholischen Medium mit vorzugsweise mindestens 10 Gewichtsprozent Wasser konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 2,5 und 8.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent bevorzugt von 1 bis 10 Gewichtsprozent enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gewichtsprozent.

Das erfindungsgemäße Mittel kann darüber hinaus die für Haarbehandlungsmittel üblichen Zusatzbestandteile enthalten, zum Beispiel Netzmittel oder Emulgatoren aus den Klassen der nichtionischen, anionischen, kationischen oder amphoteren oberflächenaktiven Tenside, wie Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,1 bis 0,5 Gewichtsprozent; Trübungsmittel, wie zum Beispiel Ethylenglykoldistearat, in einer Menge von etwa 0,2 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10 Gewichtsprozent; bakterizide und fungizide Wirkstoffe wie zum Beispiel 2,4,4-Trichlor-2-hydroxydiphenylether oder Methylchlorisothiazolion, in einer Menge von 0,01 bis 1,0 Gewichtsprozent; Verdickungsmittel, wie beispielsweise Kokosfettsäurediethanolamid, in einer Menge von etwa 0,2 bis 3,0 Gew.%, Puffersubstanzen, wie beispielsweise Natriumcitrat oder Natriumphosphat, in einer Menge von 0,1 bis 1,0 Gewichtsprozent; Anfärbestoffe, wie zum Beispiel Fluorescein Natriumsalz, in einer Menge von etwa 0,1 bis 1,0 Gewichtsprozent; Pflegestoffe, wie zum Beispiel Pflanzen- und Kräuterextrakte, Protein- und Seidenhydrolysate, Lanolinderivate, in einer Menge von 0,1 bis 5 Gew.%; physiologisch verträgliche Silikonderivate, wie zum Beispiel flüchtige oder nichtflüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; Fettalkohole, Glanzgeber, Vitamine, Weichmacher, Kämmbarkeitsverbesserer, rückfettende Agenzien und Entschäumer.

Das erfindungsgemäße Mittel kann in verschiedenen Applikationsformen Anwendung finden, wie beispielsweise als Lotion, als Non-Aerosol Sprühlotion, welches mittels einer mechanischen Vorrichtung zum Versprühen zum Einsatz kommt, als Aerosol-Spray welches mittels eines Treibmittels versprüht wird, als Aerosol-Schaum oder als Non-Aerosol Schaum, welcher in Kombination mit einer geeigneten mechanischen Vorrichtung zum Verschäumen der Zusammensetzung vorliegt, als Haarcreme, als Haarwachs, als Gel, als Flüssiggel, als versprühbares Gel oder als Schaumgel. Auch ein Einsatz in Form einer mit einem üblichen Verdicker verdickten Lotion ist möglich.

Wenn das erfindungsgemäße Haarbehandlungsmittel in Form eines Aerosolsprays vorliegt, so enthält es zusätzlich 15 bis 85 Gewichtsprozent, bevorzugt 25 bis 75 Gewichtsprozent eines Treibmittels und wird in einem Druckbehälter abgefüllt.Als Treibmittel sind beispielsweise niedere Alkane, wie zum Beispiel n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Wenn das erfindungsgemäße Haarbehandlungsmittel in Form eines versprühbaren Non-Aerosol-Haarsprays vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Haarbehandlungsmittel in Form eines Haarschaumes (Mousse) vorliegt, so enthält es mindestens eine übliche, hierfür bekannte schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Das erfindungsgemäße Mittel weist als zusätzliche Komponente eine Vorrichtung zum Verschäumen der Zusammensetzung auf. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Haarbehandlungsmittel in Form eines Haargels vorliegt, so enthält es zusätzlich mindestens eine gelbildende Substanz in einer Menge von vorzugsweise 0,05 bis 10, besonders bevorzugt von 0,1 bis 2 Gew.%. Die Viskosität des Gels beträgt vorzugsweise von 500 bis 50.000 mm².s⁻¹ (cSt), besonders bevorzugt von 1.000 bis 15.00 mm².s⁻¹ (cSt) bei 25°C (gemessen mit einem Rotationsviskosimeter RheoStress 100 der Firma Haake bei einer Temperatur von 25°C und einem Schergefälle von 0,5 bis 1400 s⁻¹).

Wenn das erfindungsgemäße Haarbehandlungsmittel in Form eines Haarwachses vorliegt, so enthält es zusätzlich wasserunlösliche Fett- oder Wachsstoffe oder Stoffe, die der Zusammensetzung eine wachsähnliche Konsistenz verleihen, in einer Menge von vorzugsweise 0,5 bis 30 Gewichtsprozent. Geeignete wasserunlösliche Stoffe sind beispielsweise Emulgatoren mit einem HLB-Wert unterhalb von 7, Silikonöle, Silikonwachse, Wachse (z.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hochmolekulare Polyethylenglykole mit einem Molekulargewicht von 800 bis 20.000, vorzugsweise von 2.000 bis 10.000 g/mol.

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gewichtsprozent, vorzugsweise 20 bis 95 Gewichtsprozent eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden.

Wenn das erfindungsgemäße Haarbehandlungsmittel in Form einer Haarcreme vorliegt, so liegt es vorzugsweise als Emulsion vor und enthält entweder zusätzlich viskositätsgebende Inhaltsstoffe in einer Menge von 0,1 bis 10 Gewichtsprozent oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1: Vergteichsversuche

Es wurden 17 Polymerlösungen untersucht. Alle untersuchten Lösungen enthalten 3 Gew.% Feststoffgehalt an Polymeren, 4,5 Gew.% Ethanol und 92,5 Gew.% Wasser. Säuregruppen enthaltende Polymere sind zu 100% mit Aminomethylpropanol neutralisiert. Die Proben E1 bis E7 enthalten jeweils nur ein einzelnes Polymer in einer Menge von 3 Gew.%. Die Proben K1 bis K10 enthalten Zweierkombinationen dieser Polymere in einer Menge von 1,5 Gew.% je Polymer, wobei die Proben K4 bis K10 nicht erfindungsgemäß und die Proben K1 und K3 erfindungsgemäß sind. Untersucht wurden die folgenden Polymere bzw. Polymerkombinationen:
E1: Aquaflex ®SF 40 (PVP/Vinyl Caprolactam/DMAPA Acrylates Copolymer)
E2: Luviset® CA 66 (VA/Crotonates Copolymer)
E3: Luvimer® 100 P (tert.-Butylacrylat/Ethylacrylat/Methacrylsäure Terpolymer)
E4: Amphomer® (Octylacrylamide/Acrylates/ Butylaminoethyl Methacrylate Copolymer)
E5: PVP K80 (Polyvinylpyrrolidone)
E6: PVP VA 64 (Polyvinylpyrrolidone/Vinylacetat Copolymer)
E7: Gaffix® 713 (Vinylcaprolactam/PVP/Dimethylaminoethyl Methacrylat Copolymer)
K1: Aquaflex® SF 40 + Luviset® CA 66
K2: Aquaflex® SF 40 + Luvimer® 100 P
K3: Aquaflex® SF 40 + Amphomer®
K4: Aquaflex® SF 40 + Gaffix® 713
K5: Luviset® CA 66 + PVP K 80
K6: Luvimer® 100 P + PVP K 80
K7: Amphomer® + PVP K 80
K8: Luviset® CA 66 + PVP VA 64
K9: Luvimer® 100 P + PVP VA 64
K10: Amphomer® + PVP VA 64

Für die Messungen wurden je 3 einheitliche, standardisierte Zählhaarsträhnen pro Probe verwendet. Eine Zählhaarsträhne besteht aus 100 Einzelhaaren (Eurohaar, 7,5 cm lang, mittlerer Durchmesser 73,36 um, Bündelgewicht 2,78 mg, Farbe 5/0 der Fa. Kerling). Die Messungen wurden an den ungebleichten Strähnen nach einer Wäsche mit einem Standardshampoo (10% Natriumlaurylethersulfat, 4% NaCI; 0,5 ml/g Haar) durchgeführt. Die bei 20°C und 65% relativer Luftfeuchtigkeit getrockneten Strähnen wurden in eine gefaltete Folie gelegt und mit Wasser gleichmäßig benetzt. Anschließend wurden je 30 µl der zu untersuchenden Festigerlösung auf die Strähnen gegeben und die Folien geschlossen. Nach 10 Minuten Einwirkzeit wurden die Strähnen erneut bei 20°C und 65% relativer Luftfeuchtigkeit über Nacht getrocknet. Für jede Probe wurden 3 Strähnen untersucht, an jeder Strähne wurden 3 Bruchkraftmessungen an verschiedenen Positionen der Strähne durchgeführt und zwar am Anfang, in der Mitte und am Ende der Strähne. Die angegebenen Messwerte sind die Mittelwerte aus 9 Messungen. Das Messprinzip für die Bruchkraftmessungen ist in Schema 1 dargestellt und beschrieben in: F. Frosch, F. Vogel, 6th International Hair Science Symposium of the German Wool Research Institute, Lüneburg, Germany (1988), German Wool Res. lnst. Publ. of Abstracts sowie in "Assessment of polymers for hair setting", Spray Technology & Marketing, Mai 1994, Seite 28.

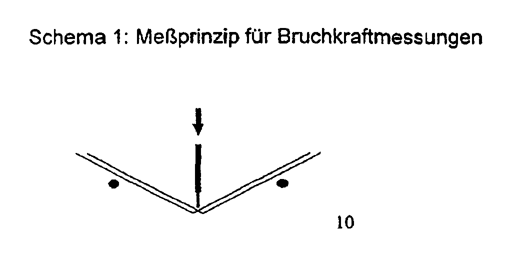

Die Messungen wurden mit einer Bruchkraft-Messapparatur durchgeführt. Hierbei liegt die Haarsträhne mit ihren Enden auf zwei Widerlagern waagerecht auf. Oberhalb der Strähne befindet sich ein mit einem Kraftsensor gekoppelter Dorn, der mit einem Motor mit konstanter Geschwindigkeit nach unten bewegt wird und dabei die Strähne zwischen den Widerlagern durchbiegt (3-Punkt-Biegeversuch). Gleichzeitig wird die Kraft, die benötigt wird, um die Haarsträhne zwischen den Widerlagern durchzubiegen, mit dem Kraftsensor (Kraftmessdose) an dem Dorn gemessen. Das Kraftsignal wird vom Prüfgerät über eine IEEE-Schnittstelle an den Steuerungsrechner übermittelt und ein Kraft-Weg-Diagramm aufgezeichnet. Die Kraft, die gebraucht wird, um die Strähne in der Mitte durchzubiegen, steigt hierbei solange kontinuierlich bis auf ein Maximum an, bis der auf dem Haar befindliche Polymerfilm bzw. die Vernetzungen der Einzelhaare untereinander aufbrechen und fällt anschließend wieder ab. Das beobachtete Maximum des Kraft-Weg-Diagramms entspricht der Bruchkraft und ist ein Maß für die Festigungsleistung der untersuchten Zusammensetzung.

Wird die Durchbiegung in gleicher Position an der Strähne wiederholt, wird eine geringere Bruchkraft gemessen, da sich einige der Polymerverbindungen zwischen den einzelnen Haarfasern irreversibel gelöst haben. Je geringer dieser Bruchkraftverlust ausfällt, um so elastischer ist der Polymerfilm bzw. um so elastischer sind die Polymerverbindungen zwischen den einzelnen Haarfasern. Die Restbruchkraft nach wiederholter Durchbiegung ist somit ein Maß für die Elastizität. Der Elastizitätsfaktor E ergibt sich aus der gemessenen Bruchkraft einer ersten Durchbiegung B1 und aus der gemessenen Bruchkraft einer zweiten Durchbiegung B2: E = B2 / B1 * 100%
Die Ergebnisse der Bruchtkraftmessungen für die erste Durchbiegung B1 und für die zweite Durchbiegung B2 sowie die daraus resultierenden Elastizitätsfaktoren E sind in Tabelle 1 dargestellt. Die angegebenen Werte für die Bruchkräfte stellen jeweils Mittelwerte von 9 Messungen dar (je drei Messungen an unterschiedlichen Positionen von 3 Strähnen).

**Tabelle 1:**

| Ergebnisse der Bruchkraft- und Elastizitätsmessungen | | | |
|---|---|---|---|
| Probe | B1 [N] | B2 [N] | E [%] |
| E1 | 0,51 | 0,19 | 37 |
| E2 | 0,31 | 0,13 | 43 |
| E3 | 0,34 | 0,13 | 37 |
| E4 | 0,52 | 0,21 | 40 |
| E5 | 0,37 | 0,17 | 46 |
| E6 | 0,24 | 0,11 | 46 |
| E7 | 0,60 | 0,21 | 35 |
| K1 | 0,27 | 0,13 | 49 |
| K2 | 0,31 | 0,14 | 45 |
| K3 | 0,37 | 0,16 | 42 |
| K4 | 0,48 | 0,18 | 36 |
| K5 | 0,35 | 0,16 | 45 |
| K6 | 0,42 | 0,16 | 37 |
| K7 | 0,58 | 0,20 | 35 |
| K8 | 0,27 | 0,11 | 43 |
| K9 | 0,34 | 0,13 | 39 |
| K10 | 0,33 | 0,12 | 37 |

Für die erfindungsgemäße Polymerkombination K1 beträgt der Elastizitätsfaktor E 49% und liegt somit deutlich über demjenigen für die Einzelpolymere E1 (37%) und E2 (43%). Für die Polymerkombination K2 beträgt der Elastizitätsfaktor E 45% und liegt somit deutlich über demjenigen für die Einzelpolymere E1 (37%) und E3 (37%). Für die erfindungsgemäße Polymerkombination K3 beträgt der Elastizitätsfaktor E 42% und liegt somit deutlich über demjenigen für die Einzelpolymere E1 (37%) und E2 (40%). In allen Fällen liegt eine synergistische Steigerung der Elastizität vor.

Für die nicht erfindungsgemäßen Polymerkombinationen K4 bis K10 liegen die Elastizitätsfaktoren jeweils entweder zwischen denjenigen für die Einzelpolymere oder sogar noch darunter. Die nicht erfindungsgemäßen Polymerkombinationen zeigen ein lediglich additives oder sogar antagonistisches Verhalten bezüglich der Elastizität.

### Beispiel 2: Haarspray

| | |
|---|---|
| 3,335 g | Aquaflex® SF 40 (PVP/Vinyl Caprolactam/DMAPA Acrylates Copolymer) |
| 3,335 g | Octylacrylamid/Acrylsäure/Butylaminoethylmethacrylat/Methylmethacrylat/Hydroxypropylmethacrylat Copolymer (Amphomer® LV 71) |
| 0,59 g | Aminomethylpropanol 95% |
| 0,20g | Parfüm |
| 0,02 g | Baysilon® Öl PD 5 |
| 10,00 g | Wasser |
| Ad 100 g | Ethanol |

Die Wirkstofflösung wurde im Verhältnis 45 : 55 mit DME als Treibmittel in eine Aerosoldose abgefüllt.

### Beispiel 3: Haarspray

| | |
|---|---|
| 3,335 g | Aquaflex ®SF 40 (PVP/Vinyl Caprolactam/DMAPA Acrylates Copolymer) |
| 3,335 g | Vinylacetat/Crotonsäure Copolymer (Luviset® CA 66) |
| 0,378 g | Aminomethylpropanol 95% |
| 0,20g | Parfüm |
| 0,02 g | Baysilon® Öl PD 5 |
| 10,00 g | Wasser |
| Ad 100 g | Ethanol |

Die Wirkstofflösung wurde im Verhältnis 45 : 55 mit DME als Treibmittel in eine Aerosoldose abgefüllt.

### Beispiel 5: Aerosol-Schaumfestiger

| | |
|---|---|
| 2,1 g | Aquaflex® SF 40 (PVP/Vinyl Caprolactam/DMAPA Acrylates Copolymer) |
| 0,6 g | Vinylacetat/Crotonsäure Copolymer (Luviset® CA 66) |
| 0,07 g | Aminomethylpropanol 95% |
| 8,9 g | Ethanol |
| 0,4 g | PEG 25 PABA |
| 0,2 g | Laureth-4 |
| 0,2 g | Panthenol |
| 0,2 g | Parfüm |
| 0,07 g | Cetyltrimethylammoniumchlorid |
| 4 g | Propan |
| 4 g | Butan |
| Ad 100 g | Wasser |

### Beispiel 6: Aerosol-Schaumfestiger

| | |
|---|---|
| 1,5 g | Aquaflex® SF 40 (PVP/Vinyl Caprolactam/DMAPA Acrylates Copolymer) |
| 0,5 g | Alkylmonoester von Polymethylvinylether/Maleinsäure Copolymer (Gantrez® ES 425) |
| 0,186 g | Aminomethylpropanol 95% |
| 8,9 g | Ethanol |
| 0,4 g | PEG 25 PABA |
| 0,2 g | Laureth-4 |
| 0,15 g | Betain |
| 0,15 g | Parfüm |
| 0,07 g | Cetyltrimethylammoniumchlorid |
| 4 g | Propan |
| 4 g | Butan |
| Ad 100 g | Wasser |

### Beispiel 7: Sprühfestiger

| | |
|---|---|
| 1,5 g | Aquaflex® SF 40 (PVP/Vinyl Caprolactam/DMAPA Acrylates Copolymer) |
| 0,45 g | Vinylacetat/Crotonsäure/Polyethylenoxid Copolymer (Aristoflex® A) |
| 27 g | Ethanol |
| 0,7 g | PEG 25 PABA |
| 0,35 g | Panthenol |
| 0,25 g | Parfüm |
| 0,21 g | PEG 40 Hydrogenated Castor Oil |
| 0,20 g | Cetyltrimethylammoniumchlorid |
| Ad 100 g | Wasser |

### Beispiel 8: Pump-Schaumfestiger

| | |
|---|---|
| 1,3 g | Aquaflex® SF 40 (PVP/Vinyl Caprolactam/DMAPA Acrylates Copolymer) |
| 0,3 g | Vinylacetat/Crotonsäure Copolymer (Luviset® CA 66) |
| 0,04 g | Aminomethylpropanol 95% |
| 8,9 g | Ethanol |
| 0,4 g | Cocamidopropyl Hydroxysultaine |
| 0,15 g | Parfüm |
| 0,1 g | Zitronensäure |
| 0,1 g | Betain |
| Ad 100 g | Wasser |

### Beispiel 9: Pump-Schaumfestiger

| | |
|---|---|
| 1,5 g | Aquaflex ®SF 40 (PVP/Vinyl Caprolactam/DMAPA Acrylates Copolymer) |
| 0,4 g | Acrylsäure/Ethylacrylat/N-tert-Butylacrylamid Copolymer (Ultrahold® 8) |
| 0,037 g | Aminomethylpropanol 95% |
| 8,9 g | Ethanol |
| 0,4 g | Cocamidopropyl Hydroxysultaine |
| 0,15 g | Parfüm |
| 0,1 g | Zitronensäure |
| 0,1 g | Betain |
| Ad 100 g | Wasser |

## Patentansprüche

1. Polymerkombination aus
(A) mindestens einem Terpolymeren aus Vinylpyrrolidon, Vinylcaprolactam und einem basischen Acrylamidmonomer und
(B) mindestens einem Polymeren mit anionischen oder anionisierbaren Gruppen,
**dadurch gekennzeichnet, dass** das Polymer (B) ausgewählt ist aus vernetzten oder unvernetzten Vinylacetat/Crotonsäure Copolymeren, Vinylacetat/Crotonsäure/Vinylalkanoat Copolymeren, Terpolymeren aus Acrylsäure, Alkylacrylat und N-Alkylacrylamid, Copolymeren aus Methylvinylether und Maleinsäuremonoalkylestern und amphoteren Polymeren.

2. Polymerkombination nach Anspruch 1, **dadurch gekennzeichnet, dass** das basische Acrylamidmonomer ausgewählt ist aus Dialkylaminoalkylmethacrylamid und Dialkylaminoalkylacrylamid, wobei die Alkylgruppen aus 1 bis 4 Kohlenstoffatomen bestehen.

3. Polymerkombination nach einem der vorstehenden Ansprüche, **dadurch gekennzeichnet, dass** es zusätzlich mindestens ein nichtionisches filmbildendes Polymer enthält.

4. Polymerkombination nach Anspruch 3, **dadurch gekennzeichnet, dass** das nichtionische filmbildende Polymer ausgewählt ist aus Polyvinylpyrrolidon und Polyvinylyrrolidon/Vinylacetat Copolymeren.

5. Haarbehandlungsmittel mit einem Gehalt an einer Polymerkombination nach einem der Ansprüche 1 bis 4 in einer geeigneten kosmetischen Grundlage.

6. Mittel nach Anspruch 5, **dadurch gekennzeichnet, dass** es in Form einer Lotion, einer Non-Aerosol Sprühlotion in Kombination mit einer mechanischen Vorrichtung zum Versprühen, in Form eines Aerosol-Haarsprays in Kombination mit einem Treibmittel, in Form eines Aerosol-Schaumes in Kombination mit einem Treibmittel oder in Form eines Non-Aerosol Schaumes in Kombination mit einer mechanischen Vorrichtung zum Verschäumen vorliegt.

7. Verwendung von Terpolymeren aus Vinylpyrrolidon, Vinylcaprolactam und einem basischen Acrylamidmonomer zur Verbesserung von Filmbildungseigenschaften oder zur Verbesserung von haarfestigenden Eigenschaften von anionischen oder anionisierbare Gruppen enthaltenden Polymeren, **dadurch gekennzeichnet, dass** die anionischen oder anionisierbare Gruppen enthaltenden Polymere ausgewählt sind aus vernetzten oder unvernetzten Vinylacetat/Crotonsäure Copolymeren, Vinylacetat/Crotonsäure/Vinylalkanoat Copolymeren, Terpolymeren aus Acrylsäure, Alkylacrylat und N-Alkylacrylamid, Copolymeren aus Methylvinylether und Maleinsäuremonoalkylestern und amphoteren Polymeren.

## Claims

1. Polymer combination of
(A) at least one terpolymer of vinylpyrrolidone, vinylcaprolactam and a basic acrylamide monomer and
(B) at least one polymer with anionic or anionizable groups,
**characterized in that** polymer (B) is selected from crosslinked or uncrosslinked vinyl acetate/crotonic acid copolymers, vinyl acetate/crotonic acid/vinyl alkanoate copolymers, terpolymers of acrylic acid, alkyl acrylate and N-alkylacrylamide, copolymers of methyl vinyl ether and maleic acid monoalkyl esters and amphoteric polymers.

2. Polymer combination according to Claim 1, **characterized in that** the basic acrylamide monomer is selected from dialkylaminoalkylmethacrylamide and dialkylaminoalkylacrylamide, where the alkyl groups consist of 1 to 4 carbon atoms.

3. Polymer combination according to one of the preceding claims, **characterized in that** it additionally comprises at least one nonionic film-forming polymer.

4. Polymer combination according to Claim 3, **characterized in that** the nonionic film-forming polymer is selected from polyvinylpyrrolidone and polyvinylpyrrolidone/vinyl acetate copolymers.

5. Hair-treatment composition with a content of a polymer combination according to one of Claims 1 to 4 in a suitable cosmetic base.

6. Composition according to Claim 5, **characterized in that** it is in the form of a lotion, of a nonaerosol spray lotion in combination with a mechanical spraying device, in the form of an aerosol hairspray in combination with a propellant, in the form of an aerosol foam in combination with a propellant or in the form of a nonaerosol foam in combination with a mechanical foaming device.

7. Use of terpolymers of vinylpyrrolidone, vinylcaprolactam and a basic acrylamide monomer for improving film-forming properties or for improving hair-setting properties of polymers, containing anionic or anionizable groups, **characterized in that** the polymers containing anionic or anionizable groups are selected from crosslinked or uncrosslinked vinyl acetate/crotonic acid copolymers, vinyl acetate/crotonic acid/vinyl alkanoate copolymers, terpolymers of acrylic acid, alkyl acrylate and N-alkylacrylamide, copolymers of methyl vinyl ether and maleic acid monoalkyl esters and amphoteric polymers..

## Revendications

1. Association de polymères constituée de
(A) au moins un terpolymère de vinylpyrrolidone, vinylcaprolactame et d'un monomère acrylamide basique et
(B) au moins un polymère à groupes anioniques ou anionisables,
**caractérisé en ce que** le polymère (B) est choisi parmi des copolymères acétate de vinyle/acide crotonique réticulés ou non réticulés, des copolymères acétate de vinyle/acide crotonique/alcanoate de vinyle, des terpolymères d'acide acrylique, d'acrylate d'alkyle et de N-alkylacrylamide, des copolymères d'oxyde de méthyle et de vinyle et de maléates de monoalkyle et des polymères amphotères.

2. Association de polymères selon la revendication 1, **caractérisée en ce que** le monomère acrylamide basique est choisi parmi un dialkylaminoalkylméthacrylamide et un dialkylaminoalkylacrylamide, les groupes alkyle étant constitués de 1 à 4 atomes de carbone.

3. Association de polymères selon l'une quelconque des revendications précédentes, **caractérisée en ce qu'**elle contient en outre au moins un polymère filmogène non ionique.

4. Association de polymères selon la revendication 3, **caractérisée en ce que** le polymère filmogène non ionique est choisi parmi la polyvinylpyrrolidone et des copolymères polyvinylpyrrolidone/acétate de vinyle.

5. Produit de traitement capillaire ayant une teneur en une association de polymères selon l'une quelconque des revendications 1 à 4, dans une base cosmétique convenable.

6. Produit selon la revendication 5, **caractérisé en ce qu'**il se trouve sous forme d'une lotion, d'une lotion à pulvériser non-aérosol, en association avec un dispositif mécanique pour la pulvérisation, sous forme d'une composition à pulvériser en aérosol en association avec un propulseur, sous forme d'une mousse pour aérosol en association avec un propulseur, ou sous forme d'une mousse non-aérosol en association avec un dispositif mécanique pour le moussage.

7. Utilisation de terpolymères de vinylpyrrolidone, vinylcaprolactame et d'un monomère acrylamide basique, pour l'amélioration des propriétés filmogènes ou pour l'amélioration des propriétés de fixage des cheveux de polymères contenant des groupes anioniques ou anionisables **caractérisé en ce que** les groupes anioniques ou anionisables contiennent des polymères qui sont choisis parmi des copolymères acétate de vinyle/acide crotonique réticulés ou non réticulés, des copolymères acétate de vinyle/acide crotonique/alcanoate de vinyle, des terpolymères d'acide acrylique, d'acrylate d'alkyle et de N-alkylacrylamide, des copolymères d'oxyde de méthyle et de vinyle et de maléates de monoalkyle, et des polymères amphotères.
